# EUROPEAN PATENT APPLICATION

(11) **EP 2 671 502 A1**
(43) Date of publication of application: **11.12.2013**
(21) Application number: 12171406.7
(22) Date of filing: 08.06.2012
(51) Int. Cl.: A61B 1/015, A61B 1/018, A61B 1/00

(54) **Fastening device for coupling the end of an endoscope to a hose**

(71) Applicant: Infobroking Limited, 302-8 Hennessy Road (HK)
(72) Inventor: Matteja, Bruno, Mercenasco (IT)
(74) Representative: Bergadano, Mirko

(57) **Abstract**

A fastening device (1) in plastic material has a sleeve (9), which can be fastened to a disposable hose (4) and defines an axial seat (10), which is a through one and is engaged, in use, by the end (2) of an endoscope (3) with an interference coupling; the sleeve (9) has at least one weakening (37) which makes the inside of the sleeve (9) elastically yielding in a circumferential direction, so as to adapt the perimeter of the seat (10) to the dimensional tolerances of the end (2) of the endoscope (3).

## Description

The present invention relates to a fastening device for coupling the end of an endoscope to a hose.

The U.S. patent application published with No. US2010/0210909 describes some solutions corresponding to the preamble of claim 1 and provided with a sleeve, which is engaged by one end of an endoscope and is coupled in a fixed position to a disposable hose. The disposable hose defines a first duct for the passage of tools with which it is possible to perform a biopsy, and a second duct for the passage of a rinsing fluid, which at the outlet is directed laterally towards a lens arranged at the end of the endoscope.

The sleeve has the purpose to maintain the end of the endoscope, with its lens, in a fixed position with respect to the hose, regarding both the axial translation and the rotation.

In most of the solutions described in the document US2010/0210909, the sleeve defines a cylindrical seat with circular cross section. The end of the endoscope is axially inserted in the seat of the sleeve. The sleeve and the end of the endoscope are dimensionally designed so as to define an interference coupling. In fact, said coupling must determine a radial forcing on the end of the endoscope, to hold the endoscope in a fixed position.

However, due to the dimensional tolerances of the endoscope, only in certain cases a stable coupling is obtained, as desired, while in other cases the interference is excessive and could damage the endoscope, or the coupling has clearance, and thus, the endoscope tends to move or even to disengage from the hose.

In one of the solutions shown in US2010/0210909, the seat of the sleeve is not circular, but its perimeter has a series of convex portions that define respective ribs. Said ribs tend to increase the interference between the endoscope and the sleeve, but may create difficult insertion of the endoscope.

The U.S. patent application published with No. US2008/0281299 describes an open clip, engaged by one end of an endoscope, but the coupling between the arms of the clip and the end of the endoscope is not stable and safe.

The purpose of the present invention is to provide a fastening device for coupling the end of an endoscope to a hose, which allows to solve in a simple and economic way the above mentioned problems.

According to the present invention a fastening device is provided for coupling the end of an endoscope to a hose, the fastening device being made of plastic material and comprising:
- a sleeve having a seat, which through-extends along an axis and comprises a first section defined by an annular surface closed and engaged, in use, by said end with an interference coupling;
- fastening means for connecting said sleeve to said hose; **characterized in that** said closed annular surface defines a weakening, which makes said sleeve elastically yielding in a circumferential direction of said first section.

The invention will now be described with reference to the accompanying drawings, which illustrate a non-limiting example of embodiment, wherein:
- Figure 1 is a perspective of a preferred embodiment of the fastening device for coupling the end of an endoscope to a hose, according to the present invention;
- Figure 2 is a different perspective of the fastening device of Figure 1;
- Figure 3 shows a section plane of the fastening device of Figures 1 and 2; and
- Figure 4 is a larger-scale section along line IV-IV of Figure 3.

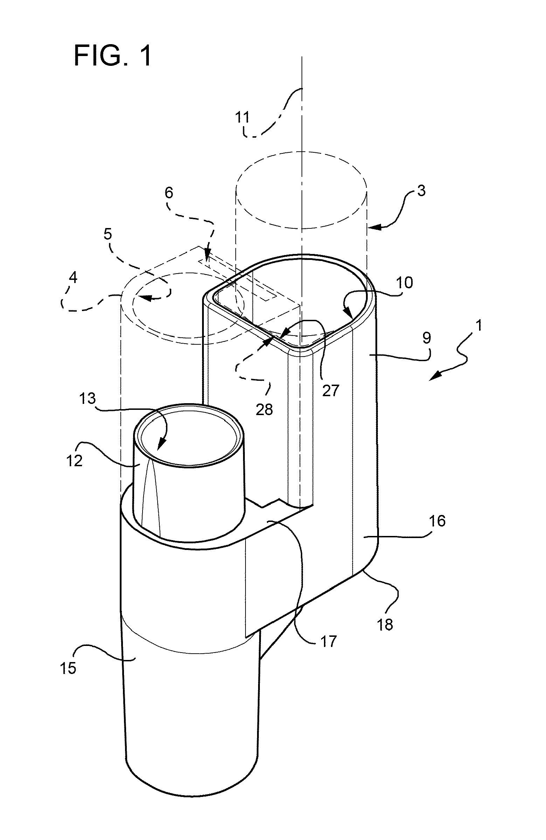

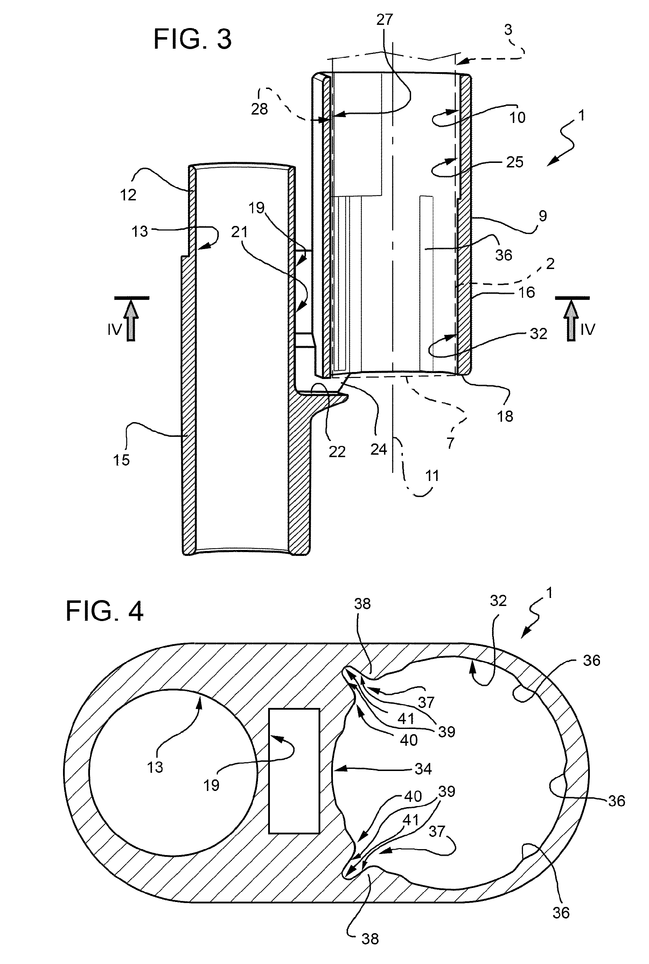

In Figures 1 and 3, with number 1 is indicated a fastening device, for coupling the end of an endoscope 3 to a hose 4. The hose 4 and the endoscope 3 are partially shown in dashed line and are made of flexible materials, e.g. plastic materials. Preferably, the hose 4 is of disposable type, and defines two ducts 5, 6: the duct 5 is used for the passage of tools provided to perform a biopsy or for the passage of catheters (not shown), while the duct 6 is used for the passage of a washing liquid for rinsing a lens 7 (in dashed line in Fig. 3) arranged on the end 2.

The device 1 is made of plastic material, in one piece or in several parts fastened together, and comprises a sleeve 9 which defines a seat 10, which extends along an axis 11 and is axially passing through. The sleeve 9, in use, is coupled in a fixed position to the hose 4 and laterally protrudes with respect to the hose 4, so as to be able to mount the endoscope 3 and the hose 4, side by side. In the illustrated embodiment, the device 1 comprises a collar 12, which engages, in a fixed position, the end of the duct 5 and defines the entrance of a hole 13.

With particular reference to Figure 3, the hole 13 is parallel to the seat 10 and defines the continuation of the duct 5. The device 1 comprises a cylindrical tip 15 which defines the final part of the hole 13, is coaxial with the collar 12, is joined to a terminal portion 16 of the sleeve 9 by means of a radial arm 17, and ends in an axial position more advanced with respect to the end edge 18 of the portion 16.

The arm 17 has an internal passage 19 which defines the extension of the duct 6 when the hose 4 is fitted over the collar 12. The passage 19 is L-shaped and comprises an inlet portion 21 parallel to the outlet axis 11 and an outlet portion 22, which is transverse, is directed towards the axis 11 and ends at the edge 18, where in use the lens 7 is approximately arranged. The sleeve 9 or the arm 17 comprise two stopping appendixes 24 arranged at the outlet of the seat 10, substantially along the edge 18, to stop the axial insertion of the end 2.

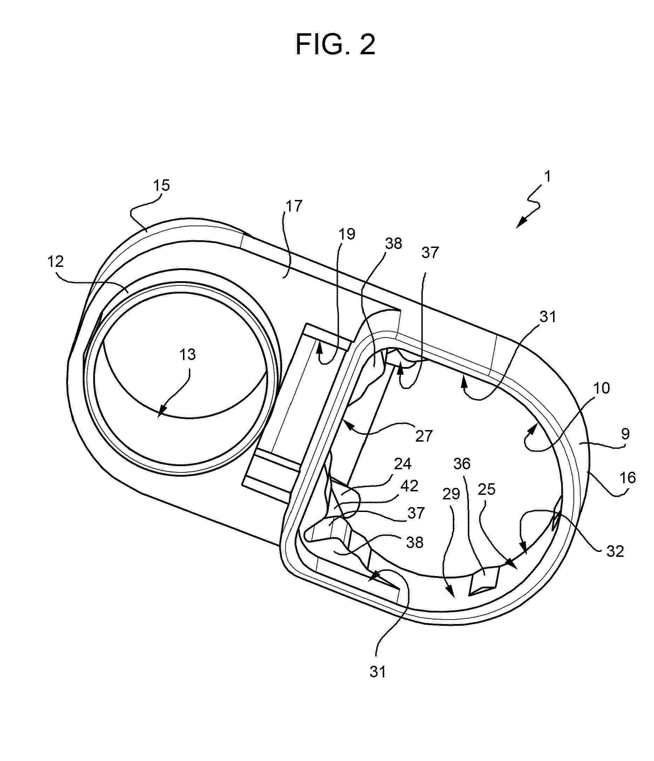

With reference to Figures 1 and 2, the seat 10 comprises an inlet section 25, having a non-circular profile. In fact, the section 25 has a flat surface 27 that is supportingly coupled to a corresponding flat face 28 of the endoscope 3, so as to maintain the endoscope 3 angularly locked about the axis 11. The section 25 has a width equal to or greater than the diameter of the end 2 of the endoscope 3, in order to easily insert the end 2 in the seat 10. In particular, the section 25 has a semi-cylindrical surface 29 facing the surface 27 and two parallel flat faces 31, which are tangent to the ends of the surface 29 and are radiused to the surface 27.

With reference to Figures 3 and 4, the seat 10 comprises a final section 32, which follows the section 25 along the axis 11, is engaged by the end 2 of the endoscope 3, and is defined by a side surface 34 having substantially the same cylindrical shape and the same outside diameter of the end 2. The surfaces 34 and 29 extend in extension of one another. At the same time, the surface 27 lies on a plane tangent to the surface 34.

With reference to Figures 2 and 4, the surface 34 is provided with a plurality of ribs 36, which are parallel to axis 10, radially project towards the inside by a relatively small amount (e.g. 0.1 mm), extend throughout the axial length of the section 32, and determine an interference coupling with the end 2 of the endoscope 3.

The surface 34 is formed by two sectors, which are diametrically opposed and are spaced along the perimeter of the section 32, by two grooves or recesses 37, which radially extend outwards with respect to the surface 34 and define a weakening.

The surface 34 and the surface of the recesses 37 constitute, together, an annular closed surface surrounding the end 2 of the endoscope 3.

The weakening provided by the recesses 37 makes the sleeve 9 elastically yielding in a circumferential direction in correspondence to the perimeter of the section 32, for which the perimeter of the section 32, i.e. said annular closed surface, can expand and adapt to the diameter of the end 2 of the endoscope 3, thus compensating for any unfavorable tolerances of said diameter.

The grooves 37 are parallel to the axis 11 and are made in respective portions 38 (Fig. 2), which axially define the bottom of the section 25. With reference to Figure 4, each groove 37 is formed by two surfaces 39, which are facing each other, are joined to the surface 34 by way of respective fittings 40, are converging to one another towards the outside, and are joined together by a bottom connector 41. The grooves 37 are axially closed by respective walls 42, having a thickness relatively small (e.g. 0.1 mm), at the edge 18 (Fig. 2).

As mentioned above, due to interference coupling, the end 2 of the endoscope 3 remains blocked by the surface 34 and by the ribs 36. At the same time, especially if the interference is excessive, the perimeter of the section 32 tends to widen, in that the inside of the sleeve 9 tends to elastically yield in the circumferential direction, in correspondence to the grooves 37. In other words, the surfaces 39 can open out slightly in an elastic way to compensate for the dimensional tolerances of the diameter of the end 2 of the endoscope 3 and can always make the coupling between the endoscope 3 and the sleeve 9, regardless of said tolerances.

At the same time, as mentioned above, the flattening defined by surface 27, being coupled to the corresponding face 28 of the endoscope 3, prevents rotation of the endoscope 3 with respect to the sleeve 9.

Other advantages of the device 1 are obvious from the characteristics described above.

From the above it is finally clear that the device 1 described can be subject to modifications and variations which do not depart from the scope of the present invention, as defined in the appended claims.

The coupling of the sleeve 9 to the hose 4 could be different from that indicated by way of example, in that the tip 15, the collar 12 and the arm 17 may be absent or different from those shown. For example, the sleeve 9 may be formed in one piece with the hose 4 or be welded to a side portion of the hose 4, whereby the fastening between the sleeve 9 and the hose 4 could be defined by the plastic material with which these two components are made.

The sleeve 9 may have a different number of grooves 37, even just one. In addition, the section 32 of the seat 10 may be defined by a surface other than the cylindrical surface 34 indicated by way of example.

The interference coupling at the section 32 could be sufficient to prevent relative rotation between the endoscope 3 and the sleeve 9, so that the section 25 could possibly be absent, in a similar way, theoretically the sleeve 9 may have only the section 25, without the section 32.

## Claims

1. - A fastening device (1) for coupling the end (2) of an endoscope (3) to a hose (4), the fastening device being made of plastic material and comprising:
- a sleeve (9) having a seat (10), which through-extends along an axis (11) and comprises a first section (32) defined by a closed annular surface and engaged, in use, by said end (2) with an interference coupling;
- fastening means (15,12,17) for connecting said sleeve (9) to said hose (4);
**characterized in that** said closed annular surface defines a weakening (37), that makes said sleeve (9) elastically yielding in a circumferential direction at said first section (32).

2. - The fastening device according to claim 1, **characterized in that** said weakening is defined by at least one recess (37), which is hollowed out radially and outwards with respect to a side surface (34) of said first section (32).

3. - The fastening device according to claim 2, **characterized in that** said recess is defined by a groove (37) extending for the entire length of said first section (32).

4. - The fastening device according to claim 3, **characterized in that** said groove (37) is defined by two surfaces (39), which converge outwards and are radiused to said side surface (34).

5. - The device according to any of claims 2 to 4, **characterized in that** said side surface (34) is a cylindrical surface.

6. - The device according to any of claims 2 to 5, **characterized in that** said weakening is defined by two recesses (37) spaced along the perimeter of said first section (32).

7. - The device according to any of claims 2 to 6, **characterized in that** said side surface (34) has a plurality of ribs (36).

8. - The device according to any of the preceding claims, **characterized in that** said seat (10) comprises a second section (25) which is next to said first section (32) along said axis (11) and has a flat surface (27) resting, in use, on a corresponding face (28) of said endoscope (3) to avoid the rotation of said end (2) with respect to said sleeve (9).

9. - The device according to claim 8, **characterized in that** said flat surface (27) is parallel to said axis (11).

10. - The device according to claim 9, **characterized in that** said second section (25) further has:
- a semi-cylindrical surface (29), and
- two faces (31), which are parallel, are tangent to the ends of said semi-cylindrical surface (29) and are radiused to the ends of said flat surface (27).

11. - The device according to claims 2 and 8, **characterized in that** said recess (37) is made in a portion (38) that axially defines said second section (25).
